# EUROPEAN PATENT APPLICATION

(11) **EP 4 338 786 A1**
(43) Date of publication of application: **20.03.2024**
(21) Application number: 23196658.1
(22) Date of filing: 11.09.2023
(51) Int. Cl.: A61M 60/139, A61M 60/295, A61M 60/531

(54) **INTRA-AORTIC BALLOON PUMP ASSEMBLY WITH PRESSURE SENSOR**

(30) Priority: 13.09.2022 US 202217944130
(71) Applicant: NuPulseCV, Inc., Raleigh, NC 27607 (US)
(72) Inventor: Woolley, Joshua Ryan, Raleigh, NC 27607 (US); Patel-Raman, Sonna Manubhai, Raleigh, NC 27607 (US); Giridharan, Guruprasad Anapathur, Raleigh, NC 27607 (US)
(74) Representative: Forresters IP LLP

(57) **Abstract**

An intra-aortic balloon pump (IABP) assembly is configured to be positioned in a patient's descending aorta to provide support to the patient. The IABP assembly includes an expandable member having a distal end and a proximal end and a driveline having a distal end and a proximal end, the distal end of the driveline is configured to be coupleable to the proximal end of the expandable member. The IABP assembly also includes a first driveline pressure sensor device disposed within the driveline that is configured to generate a first driveline pressure signal communicative of the pressure of the environment external to the driveline. The first driveline pressure sensor device includes a first driveline sensing element that is sensitive to changes in ambient pressure and positioned at or proximate to the distal end of the driveline.

## Description

### TECHNICAL FIELD

The present technology is directed to mechanical circulatory support devices and, in particular, to blood pump assemblies and associated devices, systems and methods.

### BACKGROUND

The prevalence of heart failure (HF) is increasing worldwide and is an expensive burden on health care providers. Despite advances in medical care, prognosis with HF remains poor, especially in advanced stages. Heart transplantation remains limited by the supply of donor organs. The use of left ventricular assist devices (LVAD) is stagnant at approximately 5,000 implants per year due to, among other things, the need for major operative intervention and the use of cardiopulmonary bypass (CPB). Additionally, the high cost of these devices has prevented adoption in large potential markets, with some countries deciding not to fund the use of chronic LVADs.

Globally, the most common assist device for acute heart failure is the intra-aortic balloon pump (IABP), which is used clinically for limited time periods of several hours to several days. An IABP (also referred to herein as a "blood pump," a "balloon" or an "expandable member") is part of an IABP assembly which includes a driveline with two ends. One end is coupleable to the IABP and the other end is, often indirectly, coupleable to an external drive unit. The drive unit is the source of the working fluid (e.g., ambient air or helium), which is carried via the driveline to the IABP for inflation. The drive unit is also responsible for the deflation of the working fluid from the IABP, again via the driveline. Each year more than 150,000 patients worldwide receive IABP therapy. IABPs are much simpler than current LVADs, and the therapeutic effectiveness of counterpulsation therapy is well established. Counterpulsation requires no direct cannulation of the heart leading to easier implantation and explantation. Counterpulsation therapy is also less expensive compared to LVAD therapy.

Counterpulsation therapy is achieved by rapidly inflating the balloon immediately after aortic valve closure (dicrotic notch) and rapidly deflating the balloon just before the onset of systole. The dicrotic notch may be detected using a pressure sensor disposed at the tip of the IABP and the onset of systole may be detected using an electrocardiogram (ECG). Inflation and deflation, however, may both be triggered by either pressure sensor or ECG data. An example of an IABP with a pressure sensor disposed at the tip is the Arrow Ultra 8 Fiber-Optic IAB Catheter manufactured by Teleflex. The rapid inflation of the balloon increases the diastolic aortic pressure by 20-70%, improving end-organ and coronary perfusion. The rapid deflation of the balloon reduces the ejection pressure of the native ventricle, reducing afterload and left ventricular external work. Counterpulsation therapy has been shown to be most effective in patients when their systolic aortic pressures are between 40-70 mmHg, native heart rates between 80-110 bpm, and when the counterpulsation volume (i.e., balloon volume) equals the stroke volume of the native left ventricle.

IABPs have been used in HF patients awaiting transplant and in patients undergoing coronary artery bypass surgery. The balloon is generally implanted in the descending aorta with the driveline extending through the femoral artery. This implantation being sometimes referred to herein as the "femoral implantation" and the process resulting in the femoral implantation being sometimes referred to herein as the "femoral technique." The femoral technique is a simple one as there are no significant arterial tortuosity or arterial curvature for the implanting clinician to deal with. However, IABPs implanted via the femoral technique require the patient to remain supine for the duration of therapy with the leg immobilized because (1) changes in orientation have been shown to diminish the effectiveness of therapy, (2) ambulation may cause the balloon or balloon driveline to kink due to movement of the leg leading to cyclic fatigue and ultimately failure of the balloon assembly, and (3) ambulation increases the risk of bleeding at the arterial access in the femoral artery. Consequently, patients cannot walk, or benefit from the IABP as an extended therapy for myocardial support. The lack of ambulation has been demonstrated to lead to poorer outcomes and prolong patient recovery and hospitalization. In addition to arterial access, biocompatibility, and durability issues limit the application of IABP to short durations (typically 2-4 days). While IABP support has been used for prolonged periods, the frequency of vascular complications, infections and bleeding are high.

More recently, IABPs have been implanted in the descending aorta with the driveline extending through the axillary or subclavian artery. This implantation being sometimes referred to herein as the "axillary implantation" and the process resulting in the axillary implantation being sometimes referred to herein as the "axillary technique." When the IABP is implanted using the axillary technique, certain mobility issues related to femoral implantation are mitigated, facilitating patient ambulation. Axillary implantation has typically been performed surgically via entering the chest and cutting down to the axillary/subclavian artery. However, because conventional IABPs position the pressure sensor at the tip (or distal end) of the IABP, when axillary techniques are utilized, the pressure sensor is positioned further away from the aortic arch in the abdominal aorta. This may lead to delays in detection of the dicrotic notch and balloon inflation and/or balloon deflation (e.g., if the IABP is operated without the use of ECG data), and otherwise reduced efficacy of therapy.

### BRIEF DESCRIPTION OF THE DRAWINGS

Many aspects of the present technology may be better understood with reference to the following drawings. The components in the drawings are not necessarily to scale, and instead emphasis is placed on illustrating clearly the principles of the present disclosure.
FIGs. 1A and 1B illustrate the aortic arch vasculature with a IABP having a pressure sensing element disposed at the top or distal end of the IABP, the IABP being disposed in the descending aorta using two different techniques: a femoral technique (FIG. 1A) and an axillary technique (FIG. 1B).
FIG. 2A illustrates an IABP assembly in accordance with an embodiment of the present disclosure, the IABP assembly having an expandable member, a driveline having a working fluid lumen defined by a driveline wall, a wire lumen disposed in and through the expandable member and the driveline, and a driveline pressure sensor device having a driveline pressure sensing element disposed in the working fluid lumen and located at or proximate to the distal end of the driveline.
FIG. 2B illustrates a longitudinal cross-section of the driveline segment of the IABP assembly of FIG. 2A.
FIG. 2C illustrates a transverse cross-section of the driveline segment of FIG. 2B taken along segment Z-Z of FIG. 2A.
FIG. 2D illustrates a close up of the longitudinal cross-section of the driveline segment of FIG. 2B highlighting the driveline pressure sensor device.
FIG. 3 illustrates a depiction of the aortic arch vasculature with the IABP assembly of FIGs. 2A-D disposed in the descending aorta using an axillary technique.
FIG. 4A illustrates an IABP assembly in accordance with a second embodiment of the present disclosure, the IABP assembly having an expandable member, a driveline having a working fluid lumen defined by a driveline wall, a wire lumen disposed in and through the expandable member and the driveline, and two driveline pressure sensor devices each having a driveline pressure sensing element disposed in the working fluid lumen and located at or proximate to the distal end of the driveline.
FIG. 4B illustrates a longitudinal cross-section of the driveline segment of the IABP assembly of FIG. 4A.
FIG. 4C illustrates a transverse cross-section of the driveline segment of FIG. 2B taken along segment Z-Z of FIG. 4A.
FIG. 4D illustrates a close up of the longitudinal cross-section of the driveline segment of FIG. 4B highlighting the second driveline pressure sensor device .
FIG. 5A illustrates an IABP assembly in accordance with a third embodiment of the present disclosure, the IABP assembly having an expandable member, a driveline having a working fluid lumen defined by a driveline wall, a wire lumen disposed in and through the expandable member and the driveline and further disposed in the driveline wall, and a driveline pressure sensor device having a driveline pressure sensing element disposed in the driveline wall and located at or proximate to the distal end of the driveline.
FIG. 5B illustrates a longitudinal cross-section of the driveline segment of the IABP assembly of FIG. 5A.
FIG. 5C illustrates a transverse cross-section of the driveline segment of FIG. 5B taken along segment Z-Z of FIG. 4A.
FIG. 5D illustrates a close up of the longitudinal cross-section of the driveline segment of FIG. 5B highlighting the driveline pressure sensor device.
FIG. 6A illustrates an IABP assembly in accordance with a fourth embodiment of the present disclosure, the IABP assembly having an expandable member, a driveline having a working fluid lumen defined by a driveline wall, a wire lumen disposed in and through the expandable member and the driveline and further disposed in the driveline wall, and two driveline pressure sensor devices each having a driveline pressure sensing element disposed in the driveline wall and located at or proximate to the distal end of the driveline.
FIG. 6B illustrates a longitudinal cross-section of the driveline segment of the IABP assembly of FIG. 6A.
FIG. 6C illustrates a transverse cross-section of the driveline segment of FIG. 6B taken along segment Z-Z of FIG. 6A.
FIG. 7A illustrates a longitudinal illustrates an IABP assembly in accordance with a fifth embodiment of the present disclosure, the IABP assembly having an expandable member, a driveline without a wire lumen, the driveline having a working fluid lumen defined by a driveline wall, and a driveline pressure sensor device having a driveline pressure sensing element disposed in the working fluid lumen and located at or proximate to the distal end of the driveline.
FIG. 7B illustrates a longitudinal cross-section of the driveline segment of the IABP assembly of FIG. 7A.
FIG. 7C illustrates a transverse cross-section of the driveline segment of FIG. 7B taken along segment Z-Z of FIG. 7A.
FIG. 8A illustrates an IABP assembly in accordance with a sixth embodiment of the present disclosure, the IABP assembly having an expandable member, a driveline without a wire lumen, the driveline having a working fluid lumen defined by a driveline wall, and two driveline pressure sensor devices each having a driveline pressure sensing element disposed in the working fluid lumen and located at or proximate to the distal end of the driveline.
FIG. 8B illustrates a longitudinal cross-section of the driveline segment of the IABP assembly of FIG. 8A.
FIG. 8C illustrates a transverse cross-section of the driveline segment of FIG. 8B taken along segment Z-Z of FIG. 8A.
FIG. 9A illustrates a an IABP assembly in accordance with a seventh embodiment of the present disclosure, the IABP assembly having an expandable member, a driveline without a wire lumen having a working fluid lumen defined by a driveline wall, and two driveline pressure sensor devices disposed in the driveline wall, each having a driveline pressure sensing element disposed in the working fluid lumen and located at or proximate to the distal end of the driveline.
FIG. 9B illustrates a longitudinal cross-section of the driveline segment of the IABP assembly of FIG. 9A.
FIG. 9C illustrates a transverse cross-section of the driveline segment of FIG. 9B taken along segment Z-Z of FIG. 9A.
FIG. 9D illustrates a close up of the longitudinal cross-section of the driveline segment of FIG. 9B highlighting the driveline pressure sensor devices.

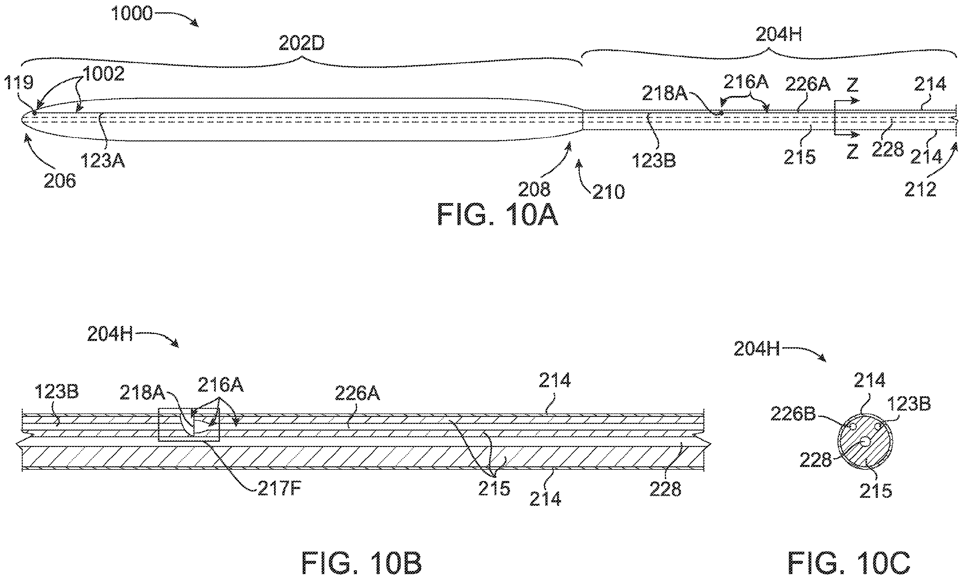

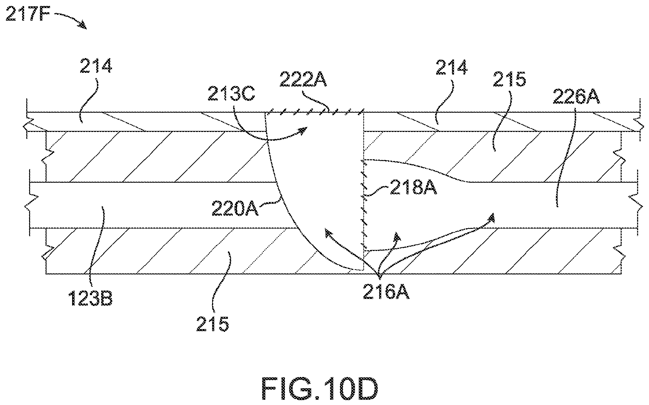

FIG. 10A illustrates an IABP assembly in accordance with an eighth embodiment of the present disclosure, the IABP assembly having an expandable member, a driveline having a working fluid lumen defined by a driveline wall, a wire lumen disposed in and through the expandable member and the driveline, a driveline pressure sensor device having a driveline pressure sensing element disposed in the working fluid lumen and located at the proximal end of the driveline, and an expandable member sensor device having an expandable member sensing element disposed at or proximate to the distal end of the expandable member.
FIG. 10B illustrates a close up of the longitudinal cross-section of the IABP assembly of FIG. 10A highlighting the driveline segment thereof.
FIG. 10C illustrates a transverse cross-section of the driveline segment of FIG. 10B.
FIG. 10D illustrates a close up of the longitudinal cross-section of the driveline segment of FIG. 10B highlighting the driveline pressure sensor device thereof.
FIG. 11A illustrates a longitudinal cross-section of an IABP assembly in accordance with a ninth embodiment of the present disclosure, the IABP assembly having an expandable member, a driveline having a working fluid lumen defined by a driveline wall, a wire lumen disposed in and through the expandable member and the driveline, and two driveline pressure sensor devices disposed in the driveline wall, both driveline pressure sensor devices located at the proximal end of the driveline.
FIG. 11B illustrates a close up of the longitudinal cross-section of the IABP assembly of FIG. 11A highlighting the driveline segment thereof.
FIG. 11C illustrates a transverse cross-section of the driveline segment of FIG. 11B.
FIG. 11D illustrates a close up of the longitudinal cross-section of the driveline segment of FIG. 11B highlighting the driveline pressure sensor devices thereof.
FIG. 12 illustrates a method of using an IABP assembly in accordance with a tenth embodiment of the present disclosure.

### DETAILED DESCRIPTION

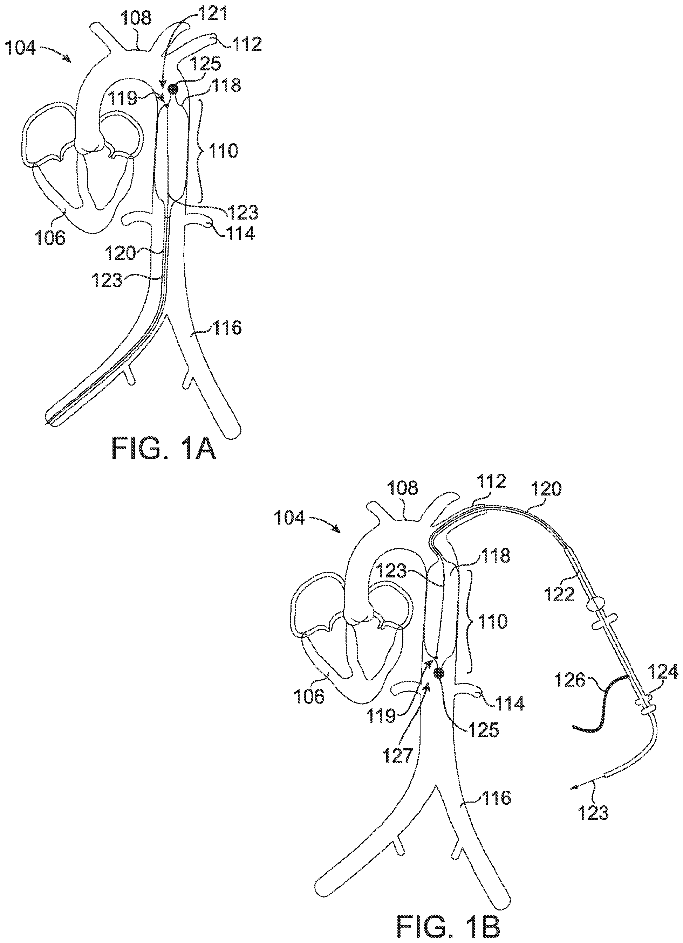

Specific details of several embodiments of the present technology are described herein with reference to FIGs. 1-12. Other applications and other embodiments in addition to those described herein are within the scope of the present technology. It should be noted that other embodiments in addition to those disclosed herein are within the scope of the present technology. Moreover, a person of ordinary skill in the art will understand that embodiments of the present technology may have configurations, components, and/or procedures in addition to those shown or described herein and that these and other embodiments may be without several of the configurations, components, and/or procedures shown or described herein without deviating from the present technology. Reference throughout this description to "one embodiment," "an embodiment," "one or more embodiments," an "nth embodiment," "some embodiments," or a phrase of similar effect means that a particular feature, structure or characteristic described in connection with the embodiment is included in at least one embodiment of the present disclosure. Thus, use of such terminology is not necessarily referring to the same embodiment. For example, it is expressly contemplated that the features described herein may be combined in any suitable manner in one or more embodiments.

### Related Technologies

Applicant/Assignee NuPulseCV, Inc. of Raleigh, North Carolina has developed a percutaneously-delivered intravascular ventricular assist system (PiVAS) that functions as a chronic counterpulsation device as generally described in U.S. Patent Application Serial No. 16/876,110, the contents of which are incorporated herein by reference in its entirety. The PiVAS includes an expandable member that is implanted in the descending aorta via an improved axillary technique using minimally invasive surgical techniques. When implanted, the driveline of the PiVAS extends through the axillary or subclavian artery. The PiVAS offers an alternative therapy for HF patients by providing partial circulatory support that may be implanted minimally invasively without entering the chest and does not require cardiopulmonary bypass (CPB) or blood products.

NuPulseCV has also developed: (1) an intra-aortic balloon pump assembly with a balloon configured for greater support, improved reinforcements, new markers, and other improvements (the "Improved Balloon Pump Assembly Technology"), generally described in a U.S. Patent Application filed on the same date as the present disclosure, entitled "Intra-Aortic Balloon Pump Assembly," identifying Joshua Ryan Woolley, Robert Christopher Hall, Duane Sidney Pinto, and Guruprasad Anapathur Giridharan as inventors, and having attorney-docket number 8019.US00/236533-30037; and (2) a blood pump support structure for a blood pump assembly (the "Blood Pump Support Structure Technology"), generally described in a U.S. Patent Application filed on the same date as the present disclosure, entitled "A Blood Pump Support Apparatus and Method for a Blood Pump Assembly," identifying Robert Christopher Hall, Joshua Ryan Woolley, Guruprasad Anapathur Giridharan, and Duane Sidney Pinto as inventors, and having attorney-docket number 8020US00/236533-30035, the contents of both applications are incorporated by reference herein in their entirety. The Improved Balloon Pump Assembly Technology solves certain problems associated with, among other things, the design of current IABPs and their drivelines, and the Blood Pump Support Structure Technology solves certain problems associated with, among other things, the blood pump rolling or folding upon itself or otherwise developing crevices when in operation (e.g., when disposed in a descending aorta).

In one embodiment, the PiVAS includes a pneumatically driven expandable member that is designed for long-term biocompatibility and safety. The proximal end of the expandable member may be coupled to a distal end of a driveline. In one embodiment, the proximal end of the expandable member may include an engagement region that is sized and shaped to fit over a distal end of the driveline. An attachment feature such as a compression ring or other suitable element providing an airtight connection/pneumatic seal between the engagement region and driveline may be used to couple the expandable member to the driveline. The proximal end of a driveline may be coupled to a drive unit (e.g., an external driver).

The IABP employed in the PiVAS may be composed of a biocompatible, non-thrombogenic elastomeric material (e.g., Biospan^{®}-S) or other suitable materials and may have features described in U.S. Patent No. 8,066,628, the disclosure of which is incorporated hereby by reference in its entirety. In some embodiments, the maximum device displacement volume may be closely matched to the stroke volume of the heart and is a parameter that may be varied to provide effective counterpulsation therapy. The expandable member may have a displacement volume between about 20 ml and about 60 ml. In some embodiments, the displacement volume is about 50 ml. The IABP may have a length between about 15 cm and about 30 cm.

The blood pump may be implanted via the femoral artery and explanted via the axillary/subclavian artery without the need to enter the chest. Regarding insertion, it may be desirable to use an introducer sheath at both the femoral and axillary/subclavian arteries. An elongated delivery dilator may be disposed in the vasculature between the two introducer sheathes (e.g., by establishing a guidewire rail and then advancing the elongated delivery dilator over the guidewire and subsequently removing the guidewire). The blood pump may be introduced into the femoral artery at the associated introducer sheath. In particular, the distal end of the elongated delivery dilator may be removably coupled to a proximal end of the driveline and the proximal end of the elongated delivery dilator may be pulled to move the blood pump into position. The elongated delivery dilator may be externalized from the axillary/subclavian artery and disconnected from the proximal end of the driveline, which may be also externalized. During introduction of the blood pump into the introducer sheath at the femoral artery, the blood pump may be folded or rolled prior to insertion. A delivery sheath (e.g., a funnel or folding tube) may be utilized to reduce a dimension of the blood pump prior to insertion. The blood pump may be non-obstructive and may lay at least substantially flat in the descending aorta without folds or crevices when deflated. This enables the device to be turned off for prolonged periods. Patients may routinely stop the device for 60-plus minutes with no adverse consequences. The blood pump has been demonstrated to have a durability for over 2.5 years of use.

The driveline may be a thin (e.g., 4.2 mm outer diameter) driveline that shuttles a working fluid (e.g., air, gas, etc.) between the blood pump and the drive unit. The driveline may include an inner driveline and an outer driveline. The inner driveline may be an elongated support structure having a lumen extending there through for delivering working fluid to and from the expandable member. The inner driveline may be positioned at least partially within the patient's vasculature (e.g., between the aorta and the axillary/subclavian artery). After implantation, the inner driveline may have a distal end portion coupled to the expandable member and positioned within the patient's vasculature (e.g., the descending aorta) and a proximal end portion coupled to a distal end of the outer driveline and positioned external to the patient's vasculature at an arteriotomy in, for example, the axillary/subclavian artery, or other suitable blood vessel. The proximal end portion of the inner driveline may be coupled to a distal end of the outer driveline using any suitable technique (e.g., compression rings, suturing, gluing, stitching, etc.).

An arterial interface device or stopper device ("AID") may be used to provide hemostasis at an arteriotomy where the inner driveline exists the vasculature. The AID may enable long-term implant using minimally invasive surgical techniques. The AID may include one or more anchoring elements used to secure the device in a desired orientation or position, and one or more ports. For example, one port might provide wire axis to the patient's vasculature. The AID may have features similar to those described in U.S. Patent No. 7,892,162, the disclosure of which is incorporated herein by reference in its entirety. The AID may be deployed or advanced over the inner driveline (e.g., that portion that is externalized from the vasculature) through a shaft in the AID that defines a lumen. The inner driveline may be inserted into and extend through the shaft. The outer driveline may be coupled to the inner driveline at a position proximate the AID.

The outer driveline may also be an elongated support structure having a lumen extending there through. The outer driveline may be positioned at least partially subcutaneously but external to the patient's vasculature. After implantation, the proximal end portion of the outer driveline may be coupled to a skin interface device (described below). The lumen of the driveline (e.g., inner and outer drivelines) may be coupled to the lumen of the expandable member to transport the working fluid to and from the expandable member. The relatively small diameter of the driveline compared to the relatively large axillary/subclavian artery mitigates risk of limb ischemia.

The drive unit may be a small, portable device that actuates the balloon pump by generating a flow of working fluid (e.g., a gas or other fluid such as ambient air or helium) into and out of the expandable member via the driveline. For example, the drive unit may generate a positive pressure to accelerate the working fluid into the expandable member, thereby inflating the expandable member and may induce a negative pressure to withdraw the working fluid from the expandable member, thereby deflating expandable member. The drive unit may utilize a bellows, a blower, a compressor, an accelerator, or other similar features to direct the flow of working fluid into and out of the expandable member. The drive unit may have a feature to prevent over-inflation of the balloon.

The skin interface device ("SID") may be a transcutaneous device that enables the drive unit to drive operation of the expandable member. The SID may provide a stable and/or secure exit site for the driveline (e.g., the outer driveline). In an embodiment where the driveline comprises an inner driveline and outer driveline, the proximal end of the outer driveline may be coupled to an internal facing portion of SID and the drive unit may be coupled to an external facing portion of the SID. As such, SID may direct gases received from the drive unit to the outer driveline for delivery to the expandable member. The SID may be similar to the interface devices described in U.S. Patent No. 10,137,230, the disclosure of which is incorporated herein by reference in its entirety.

The IABP may be triggered, at least in part, by pressure sensor data (e.g., from a pressure sensor element 119 located at the distal tip of the IABP 118, as generally depicted in FIG. 1B) and/or the patient's ECG via surface ECG sensors. The associated ECG sensors may be coupled to the SID via ECG leads, which relay the measurements received from the sensors to the drive unit via a wired or wireless connection. In other embodiments, the ECG sensors may be wirelessly connected to the drive unit and may transmit the sensed measurements directly to the drive unit without using the SID. The ECG sensors may be implanted, external or both implanted and external. For example, the ECG sensors may be implanted bipolar electrodes positioned at and/or proximate the heart or other appropriate tissue to determine, for example, when the left ventricle is contracting or relaxing. Counterpulsation therapy may be achieved by rapidly inflating the balloon in the aorta immediately after aortic valve closure (dicrotic notch) and rapidly deflating the balloon just before the onset of systole using a drive unit. The dicrotic notch may be sensed by the pressure sensor, and the onset of systole may be predicted or sensed using the ECG signals.

The rapid inflation of the balloon may increase the diastolic aortic pressure, improving end-organ perfusion and coronary perfusion. The rapid deflation of the balloon reduces the ejection pressure of the native ventricle, reducing afterload and left ventricular external work. This embodiment of the PiVAS provides counterpulsation therapy in patients that is more effective than a 40-ml IABP device due to a larger displacement volume. The PiVAS has enhanced durability, has a reduced or eliminated risk of being thrombogenic and/or obstructive, and is overall a low-cost, and less invasive device implant/explant procedure without the need to enter the chest. The PiVAS also has low serious adverse event (AE) burden, and enables non-obligatory support to a less-sick heart failure population (the device may be 'on or off' as needed).

### IABP Embodiments

FIGs. 1A and 1B illustrate the aortic arch vasculature 104 with an IABP 118 having a pressure sensing element 119 disposed at the tip or distal end of the IABP 118, the IABP 118 having been disposed in the descending aorta 110 using two different techniques: a femoral technique (as depicted in FIG. 1A) and an axillary technique (whether conventional or PiVAS) (as depicted in FIG. 1B). In both FIGs. 1A and 1B, the IABP 118 is depicted as a longitudinal cross-section coupled to driveline 120 where a pressure signal line 123 is any medium capable of transmitting a pressure signal from pressure sensing element 119. In one embodiment, pressure signal line 123 is disposed throughout the IABP 118 and driveline 120. One or more radiopaque markers 125 may optionally be included as part of IABP 118 and disposed near the distal end of IABP 118. Similarly, a wire lumen may optionally be included as part of IABP 118 and driveline 120 and may extend throughout the IABP 118 and driveline 120.

The depicted aortic arch vasculature 104 includes the heart 106, the aortic arch 108, the axillary/subclavian artery 112, the descending aorta 110, the two branches of the renal artery 114 and the two branches of the common iliac artery 116. Pressure sensing element 119 may be a diaphragm associated with a pressure sensor device that further includes a pressure signal line 123. The pressure sensor device may be a solid state, fluid-filled, gas-filled, or fiber optic pressure sensor. A solid state pressure sensor device may contain a signal transducer and a MEMs sensor that flexes with pressure. A fluid-filled pressure sensor device may translate pressure along the length of the fluid medium to a pressure transducer located external to the body. A fiber optic pressure sensor may be use, for example, a Fabry-Perot interferometer (FBI) or fiber Bragg grating (FBG).

Pressure signal transmitted by pressure signal line 123 may be a light wave, a fluid, an electric analog or digital signal, or any other media that is capable of communicating pressure information. For example, pressure signal line 123 may be a fiber optic signal line capable of acting as a light conveyer between the pressure sensing element 119 and/or an associated sensor cavity (not depicted) and a photodiode or other light sensor (not depicted). Light introduced into the pressure signal line 119 by a light source (e.g., a light emitting diode), not depicted, may be directed toward the pressure sensing element 119 and/or sensor cavity and is reflected back along the fiber optic line 119 where it is detected by a photodiode or other light sensor. Using a interferometer or other processing module, a distance (e.g., a sensor cavity length) may be determined. A change in pressure at the pressure sensing element 119 may cause the sensing element 119 to deflect or deform, changing the length of the sensor cavity length, which may be determined by re-introducing light into the fiber optic line 119, detecting the reflection and using an interferometer or other processing module. In a non-limiting example, a pressure variation (ΔP) may be derived using the formulation ΔP= ΔL/S, where (ΔL) is the difference in cavity length and S is a gauge factor derived from factory calibration. Some of these principals are generally described in U.S. Patent Nos. 3,349,623; 5,202,939; 5,392,117; and 7,229,403 and EP 1764124B1, the contents of each are expressly incorporated herein by reference. In this embodiment, the reflected light may be the pressure signal. Alternatively, pressure signal line 119 may be a tubing (e.g., a polyethylene (PE) tubing) in communication with a fluid-filled cavity and capable of communicating the pressure-sensitive fluid from the fluid to a transducer. In this embodiment, the pressure information may be the pressure-sensitive fluid. Alternatively, pressure signal line 119 may be a wire lead or wireless path and an electric analog or digital signal may be the pressure signal. If pressure signal line 118 is a wireless path, it need not travel as depicted through the IABP 118 and driveline 120, but may rather travel wirelessly "as the crow flies" to a transducer or processing module.

An axillary implantation may include the use of sheath 122, central lumen 124 and working fluid connection 126 coupled to a drive unit. As noted in the Background section of this document, during femoral implantation of the IABP 118 as depicted in FIG. 1A, the pressure sensing element 119 is positioned in a region 121 that is proximate to the aortic valve of the heart 106. This minimizes the time needed to sense the pressure waves caused by the aortic valve closure (i.e., dicrotic notch), which is a landmark for inflation of the IABP 118. But during axillary implantation of the IABP 118, the pressure sensing element 119 is positioned in a region 127 that is located farther away from the aortic valve as generally depicted in FIG. 1B. This leads to a delay in sensing the closure of the aortic valve, which results in delayed IABP 118 inflation. It may also lead to delays in deflation. Accordingly, it may reduce device efficiency.

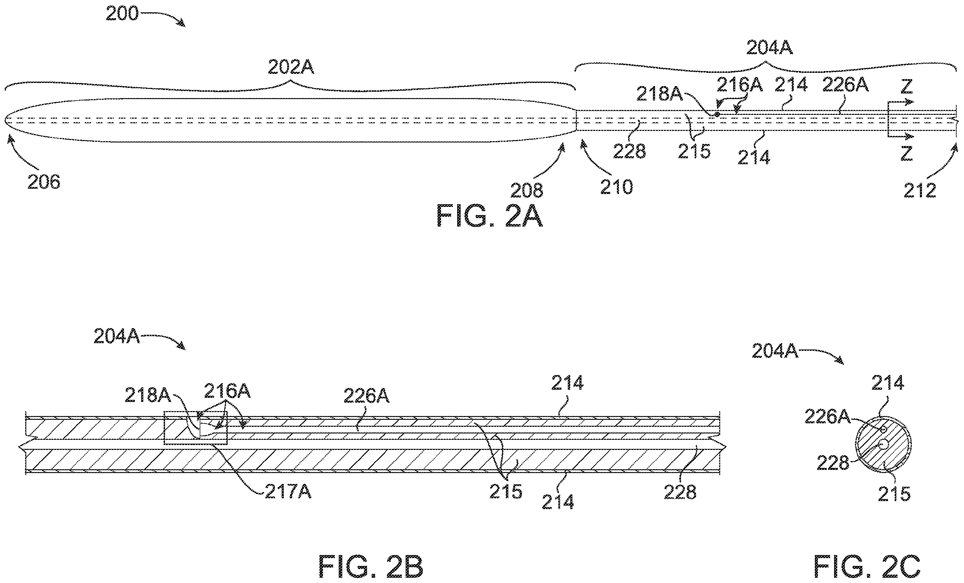

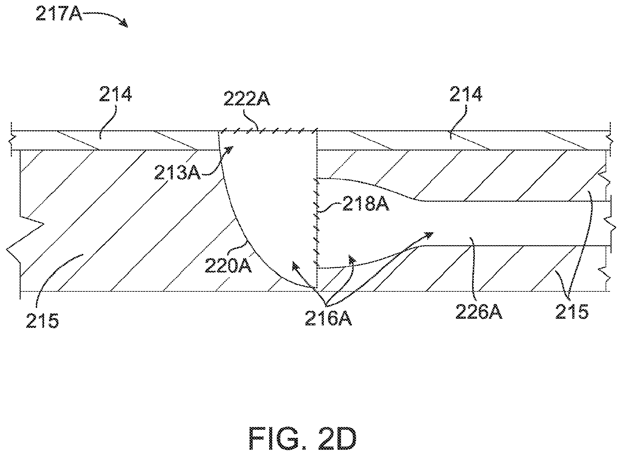

FIG. 2A illustrates an IABP assembly 200 in accordance with an embodiment of the present disclosure, the IABP assembly 200 having an expandable member 202A, a driveline 204A having a working fluid lumen 215 defined by a driveline wall 214, a wire lumen 228 disposed in and through the expandable member 202A and the driveline 204A, and a driveline pressure sensor device 216A having a driveline pressure sensing element disposed in the working fluid lumen 215 and located at or proximate to the distal end 210 of the driveline 204A. FIG. 2B illustrates a longitudinal cross-section of the driveline segment 204A of the IABP assembly 200 of FIG. 2A. FIG. 2C illustrates a transverse cross-section of the driveline segment 204A of FIG. 2B taken along segment Z-Z of FIG. 2A. FIG. 2D illustrates a close up of the longitudinal cross-section of driveline segment 204A of FIG. 2B taken at region 217A highlighting the driveline pressure sensor device 216A.

With reference to FIGs. 2A-2D, the expandable member 202A may have a distal end 206 and a proximal end 208, and the driveline 204A may have a driveline wall 214, and two ends: a distal end 210 and a proximal end 212. The proximal end 208 of expandable member 202A is capable of being coupled to the distal end 210 of driveline 204A as was described above in regard to the PiVAS. The first driveline pressure sensor device 216A may comprise a first driveline pressure sensing element 218A that is positioned at or proximate to the distal end 210 of the driveline 204A. The driveline 204A may have a working fluid lumen 215 defined by driveline wall 214, the working fluid lumen 215 may be configured to transport a working fluid such as helium or ambient air for at least one of inflation and deflation of the expandable member 202A.

A wire lumen 228 may be optionally disposed in and through the expandable member 202A and driveline 204A. The wire lumen 228 may be generally positioned at the center of the expandable member 202A and driveline 204A. Other positions of the wire lumen 228 are expressly contemplated hereby. For example, the wire lumen 228 may be positioned off center of either or both of the expandable member 202A and driveline 204A. The wire lumen 228 may be configured as a guardwire rail and sized such that a guidewire may be threaded through said wire lumen. This may facilitate implantation using an over-the-wire technique and enable easier implant through a single vascular access point.

The first driveline pressure sensor device 216A may be a fiber optic pressure sensor (e.g., based on micro-optical mechanical systems) of the type manufactured by OPsens. Alternatively, first driveline pressure sensor device 216A may be a solid-state or fluid or gas filled pressure sensor device, or any other suitable pressure sensor. The first driveline pressure sensor device 216A may be configured to generate a first driveline pressure signal communicative of (e.g., it may indicate or be representative of) the pressure of the environment external and proximate to the distal end 210 of the driveline 204A. The first driveline pressure signal may be a light wave, a fluid, an electric analog or digital signal, or any other media that is capable of communicating pressure information.

First driveline pressure sensor device 216A may include a first driveline sensor cavity 220A, a first driveline sensor window 222A, and the first driveline sensing element 218A. The first driveline sensing element 218A may be sensitive to changes in ambient pressure and may be configured as a diaphragm or a thin membrane that is capable of being deflected by ambient pressure. In one embodiment the first driveline sensing element 218A may be a micromachined silicon diaphragm membrane.

The driveline wall 214 may further include a first recess 213A extending inward relative to the exterior of the driveline 204A and located proximate to the distal end 210 of the driveline 204A. The first recess 213A may be a gap or interruption in the driveline wall 214. The first recess 213A may have a shape that at least partially houses the first driveline sensor cavity 220A. The first driveline sensor window 222A may be shaped and sized to plug the first recess 213A and may be disposed along the first recess 213A to close the first driveline sensor cavity 220A. The first driveline sensing element 218A may define a portion of a wall of the first driveline sensor cavity 220A. As depicted the first driveline sensing element 218A may be positioned in the working fluid lumen 215. Optionally, the first driveline sensing element 218A may be positioned in the driveline wall 214 or partially within both the working fluid lumen 215 and the driveline wall 214.

The first driveline sensor device 216A may further include a flexible substance (not depicted) disposed in the first driveline sensor cavity 220A. The flexible substance 224 may be operative to communicate pressure to the first driveline sensing element 218A. The flexible substance may be a gel, a fluid, a gas, and/or an elastomer (e.g., the flexible substance may be an inert gas, or a silicone gel). Alternatively, the first driveline sensor cavity 220A may constitute a vacuum. The first driveline sensor window 222A may be configured to prevent the flexible substance from leaking out of the first driveline sensor cavity 220A or to maintain the vacuum when the IABP assembly 200 is in operation.

The first driveline pressure sensor device 216A may include a first driveline pressure signal line 226A that is in communication with the first driveline sensing element 218A. The first driveline pressure signal line 226A may be disposed within the driveline 204A (i.e., within the driveline wall 214, within the working fluid lumen 215, or partially within both the driveline wall 214 and the working fluid lumen 215). The first driveline pressure signal line 226A may be configured to transmit the first driveline pressure signal that is communicative of (e.g., it may indicate or be representative of) the pressure of the environment external and proximate to the distal end 210 of the driveline 204A. The first driveline pressure signal line 226A may be a fiber optic line or any other suitable signal line capable of transmitting first driveline pressure sensor data. In another embodiment, driveline pressure signal line 226A may be a tubing (e.g., a polyethylene (PE) tubing) or a wireless connection (in communication with a MEMS-based wireless sensing element).

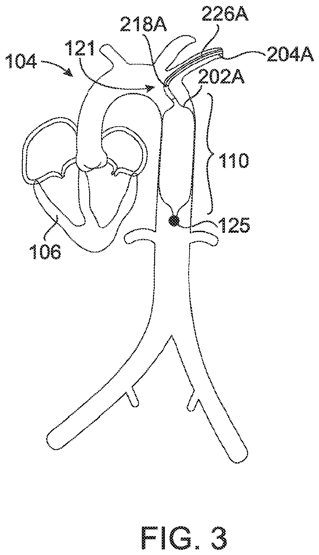

FIG. 3 illustrates a depiction of the aortic arch vasculature 104 with the IABP assembly 200 of FIGs. 2A-D disposed in the descending aorta 110 using an axillary technique. The first driveline sensing element 218A is configured to sense the pressure in region 121 and to generate first driveline pressure signal communicative of (e.g., it may indicate or be representative of) that pressure. Region 121 is external and proximate to the distal end 210 of the driveline 204A and proximate to the aortic valve of the heart 106.

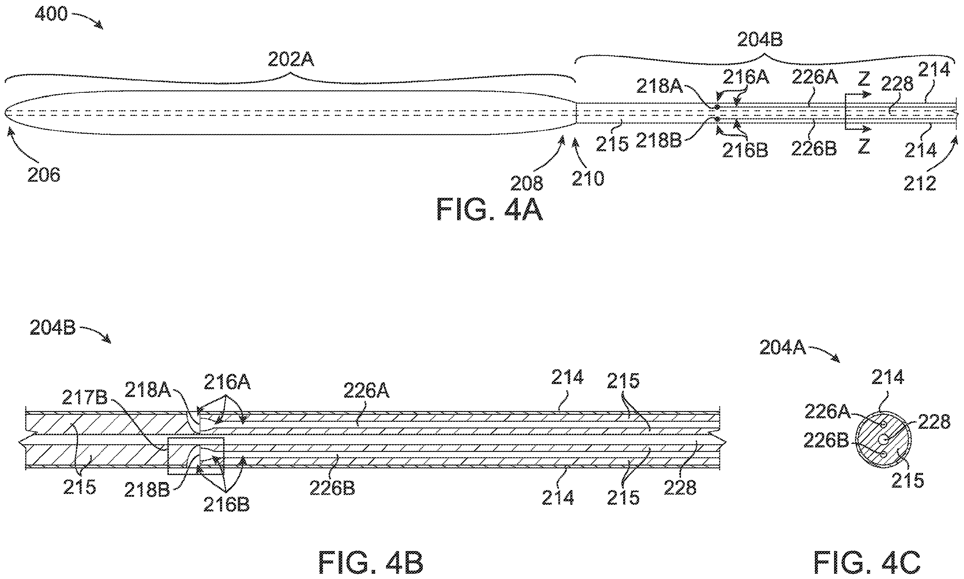

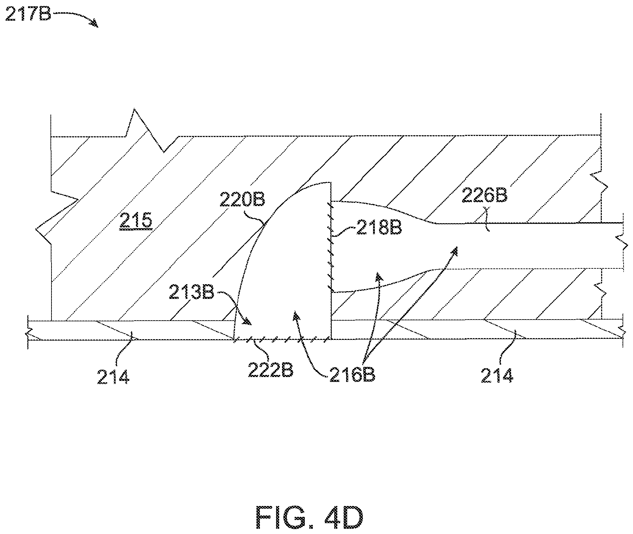

FIG. 4A illustrates an IABP assembly 400 in accordance with a second embodiment of the present disclosure, the IABP assembly 400 having expandable member 202A, a driveline 204B having a working fluid lumen 215 defined by a driveline wall 214, a wire lumen 228 disposed in and through the expandable member 202A and the driveline 204B, first driveline pressure sensor device 216A and a second driveline pressure sensor device 216B, each having a driveline pressure sensing element 218A, 218B (respectively) disposed in the working fluid lumen 215 and located at or proximate to the distal end 210 of the driveline 204A. FIG. 4B illustrates a longitudinal cross-section of the driveline segment 204B of the IABP assembly 400 of FIG. 4A. FIG. 4C illustrates a transverse cross-section of the driveline segment 204B of FIG. 4B taken along segment Z-Z of FIG. 4A. FIG. 4D illustrates a close up of the longitudinal cross-section of the driveline segment 204B of FIG. 4B taken at region 217B and highlighting the second driveline pressure sensor device 216B.

The second driveline pressure device 216B may be a fiber optic pressure sensor (e.g., based on micro-optical mechanical systems), a solid-state or fluid or gas filled pressure sensor device, or any other suitable pressure sensor. The second driveline pressure device 216B may be configured to generate a second driveline pressure signal communicative of (e.g., it may indicate or be representative of) the pressure of the environment external and proximate to the distal end 210 of the driveline 204A. The second driveline pressure signal may be: a light wave, a fluid, an electric analog or digital signal, or any other media that is capable of communicating pressure information.

FIGs. 4A-D include all of the features of FIGs. 2A-D, but adds a second recess 213B and a second driveline pressure sensor device 216B. Second driveline pressure sensor device 216B may include a second driveline sensor cavity 220B, a second driveline sensor window 222B, and the second driveline sensing element 218B. The second driveline sensing element 218B may be sensitive to changes in ambient pressure and may be configured as a diaphragm or a thin membrane that is capable of being deflected by ambient pressure. In one embodiment the second driveline sensing element 218B may be a micromachined silicon diaphragm membrane.

The driveline wall 214 may further include second recess 213B extending inward relative to the exterior of the driveline 204B and located proximate to the distal end 210 of the driveline 204B. The second recess 213A may be a gap or interruption in the driveline wall 214. The second recess 213B may have a shape that at least partially houses the second driveline sensor cavity 220B. The second driveline sensor window 222B may be shaped and sized to plug the second recess 213B and may be disposed along the second recess 213B to close the second driveline sensor cavity 220B. The second driveline sensing element 218B may define a portion of a wall of the second driveline sensor cavity 220B. As depicted, the second driveline sensing element 218B may be positioned in the working fluid lumen 215. Optionally, the second driveline sensing element 218B may be positioned in the driveline wall 214 or partially within both the working fluid lumen 215 and the driveline wall 214.

The second driveline sensor device 216B may further include a flexible substance (not depicted) disposed in the second driveline sensor cavity 220B. The flexible substance 224 may be operative to communicate pressure to the second driveline sensing element 218B. The flexible substance may be a gel, a fluid, a gas, and/or an elastomer (e.g., the flexible substance may be an inert gas, or a silicone gel). Alternatively, the second driveline sensor cavity 220B may constitute a vacuum. The second driveline sensor window 222B may be configured to prevent the flexible substance from leaking out of the second driveline sensor cavity 220B or to maintain the vacuum when the IABP assembly 200 is in operation.

The second driveline pressure sensor device 216B may include a second driveline pressure signal line 226B that is in communication with the second driveline sensing element 218B. The second driveline pressure signal line 226B may be disposed within the driveline 204B (i.e., within the driveline wall 214, within the working fluid lumen 215, or partially within both the driveline wall 214 and the working fluid lumen 215). The second driveline pressure signal line 226B may be configured to transmit the second driveline pressure signal that is communicative of (e.g., it may indicate or be representative of) the pressure of the environment external and proximate to the distal end 210 of the driveline 204B. The second driveline pressure signal line 226B may be a fiber optic line or any other suitable signal line capable of transmitting second driveline pressure sensor data. In another embodiment, driveline pressure signal line 226A may be a tubing (e.g., a polyethylene (PE) tubing) or a wireless connection (in communication with a MEMS-based wireless sensing element). The second driveline pressure signal may be: an analog signal, a digital signal, information, data, a wave (e.g., a pressure wave, a light wave, etc.), etc.

As depicted in FIGs. 4B-D, wire lumen 228 may be generally positioned at the center of the expandable member 202A and driveline 204B. Other positions of the wire lumen 228 are expressly contemplated hereby. For example, the wire lumen 228 may be positioned off center of either or both of the expandable member 202A and driveline 204B. Similarly, first and second driveline pressure sensor devices 216A and 216B may be disposed on opposing sides of the driveline 204B and in the same plane as the wire lumen 228. In one embodiment, the first and second driveline pressure sensor devices 216A and 216B may be disposed proximate to opposing ends of the driveline wall 214. Other arrangements of the first and second driveline pressure sensor devices 216A and 216B and associated first and second driveline pressure signal lines 226A and 226B are expressly contemplated and within the scope of the present disclosure.

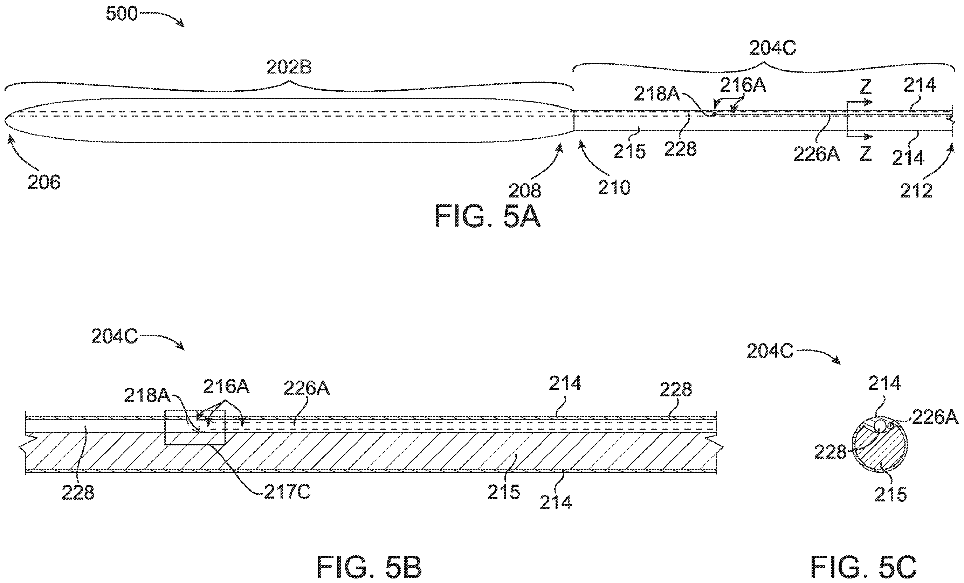

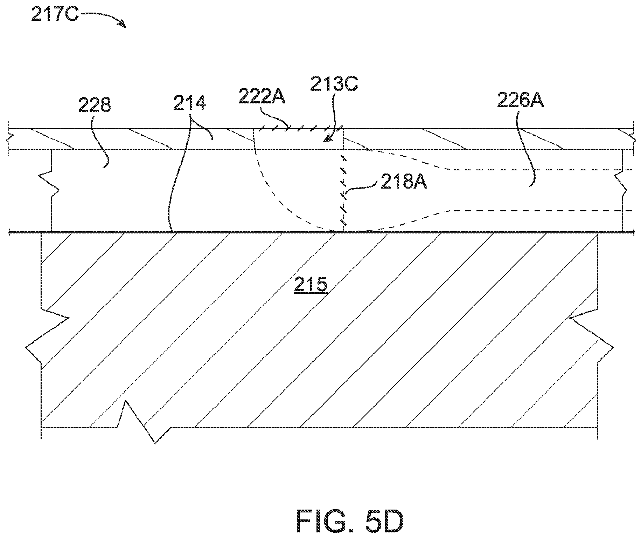

FIG. 5A illustrates an IABP assembly 500 in accordance with a third embodiment of the present disclosure, the IABP assembly 500 having expandable member 202B, a driveline 204C having a working fluid lumen 228 defined by a driveline wall 214, a wire lumen 228 disposed in and through the expandable member 202A and the driveline 204C and further disposed in the driveline wall 214, and a driveline pressure sensor device 216A having a driveline pressure sensing element disposed in the driveline wall 214 and located at or proximate to the distal end 210 of the driveline 204C. FIG. 5B illustrates a longitudinal cross-section of the driveline segment 204C of the IABP assembly 500 of FIG. 5A. FIG. 5C illustrates a transverse cross-section of the driveline segment 204C of FIG. 5B taken along segment Z-Z of FIG. 4A. FIG. 5D illustrates a close up of the longitudinal cross-section of the driveline segment 204C of FIG. 5B highlighting the driveline pressure sensor device 216A.

FIGs. 5A-D include all of the features of FIGs. 2A-D, with the exception that the IABP assembly 500 positions the wire lumen 228 and the first driveline pressure device 216A (inclusive of the first driveline pressure signal line 226A) in the driveline wall 214. In one embodiment, the first driveline pressure device 216A (inclusive of the first driveline pressure signal line) and the wire lumen 228 are disposed in the same plane. Other arrangements are expressly contemplated and within the scope of the present disclosure. In one embodiment, the driveline wall 214 is of uniform thickness. In one embodiment, the driveline wall 214 has thickness that varies to accommodate the wire lumen 228 and either or both of the first driveline pressure device 216A and the first driveline pressure signal line 226A. As depicted, the recess 213A has a shape that houses the first driveline sensor device 216A.

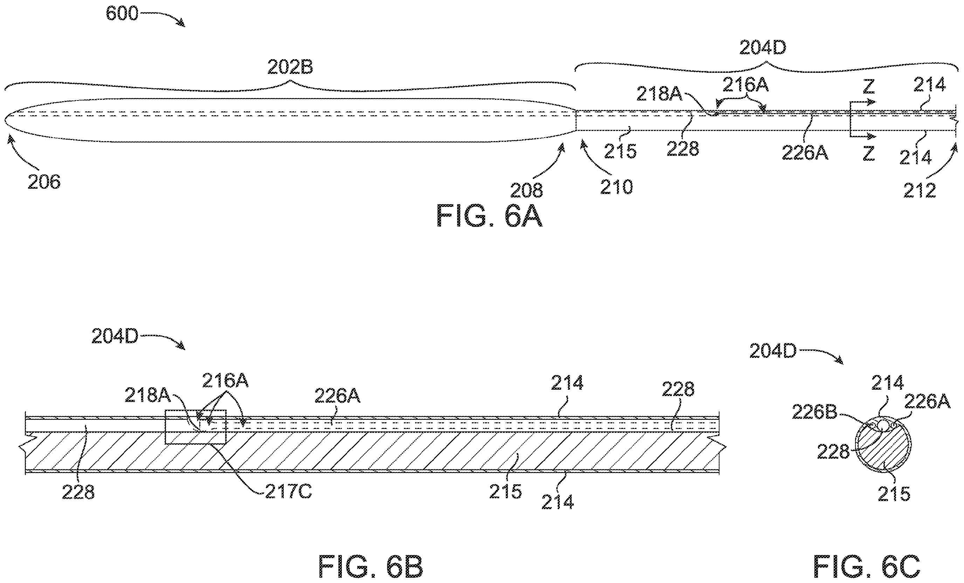

FIG. 6A illustrates an IABP assembly 600 in accordance with a fourth embodiment of the present disclosure, the IABP assembly 600 having expandable member 202B, a driveline 204D having a working fluid lumen 215 defined by a driveline wall 214, a wire lumen 228 disposed in and through the expandable member 202A and the driveline 204D and further disposed in the driveline wall 214, and two driveline pressure sensor devices 216A and 216B, each having a driveline pressure sensing element 218A, 218B (respectively) disposed in the driveline wall 214 and located at or proximate to the distal end 210 of the driveline 204D. FIG. 6B illustrates a longitudinal cross-section of the driveline segment 204D of the IABP assembly 600 of FIG. 6A. FIG. 6C illustrates a transverse cross-section of the driveline segment 204D of FIG. 6B taken along segment Z-Z of FIG. 6A. FIGs. 6A-C are identical to FIGs. 5A-C with the exception that a second driveline pressure device 216B is further disposed in the driveline wall 214. In one embodiment, the second driveline pressure device 216B is positioned in the same plane as the wire lumen 228 and the first driveline pressure device 216A. Other arrangements are expressly contemplated and within the scope of the present disclosure.

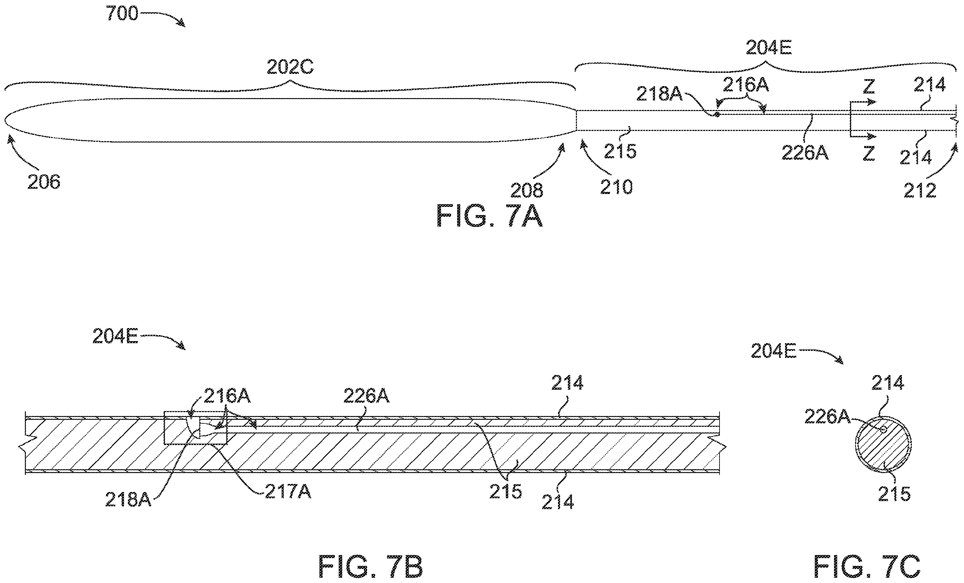

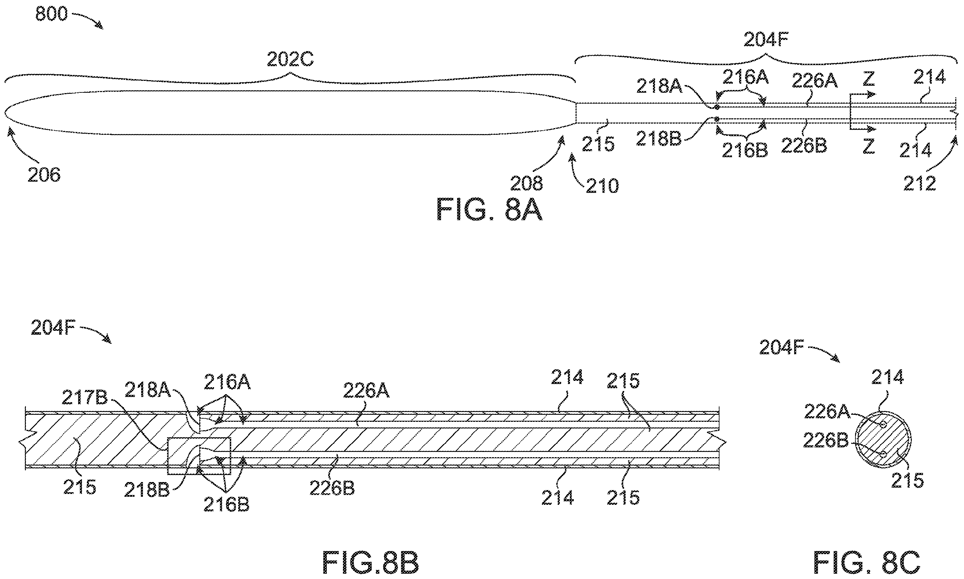

FIGs. 7A-C are identical to FIGs. 2A-C and FIGs. 8A-C are identical to FIGs. 4A-C, but in the embodiments of FIGs. 7A-C and 8A-8C, the wire lumen 228 is omitted. To differentiate the embodiments, the embodiment of FIGs. 7A-C includes an IABP assembly 700, expandable member 202C and a driveline 204E. And the embodiment of FIGs. 8A-C includes an IABP assembly 800, expandable member 202C and a driveline 204F. Driveline wall 214 may have a uniform thickness. In other embodiments, the driveline wall 214 has a varied thickness.

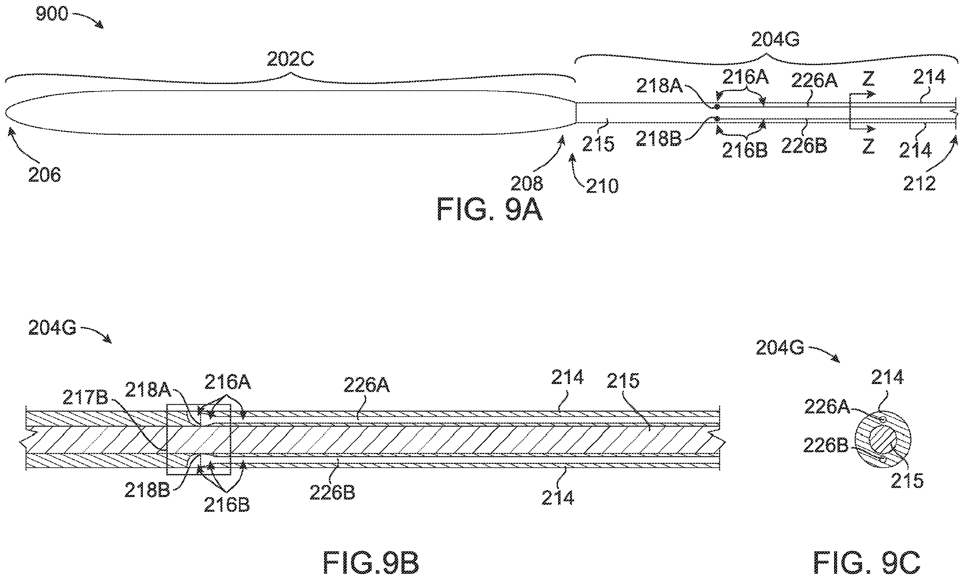

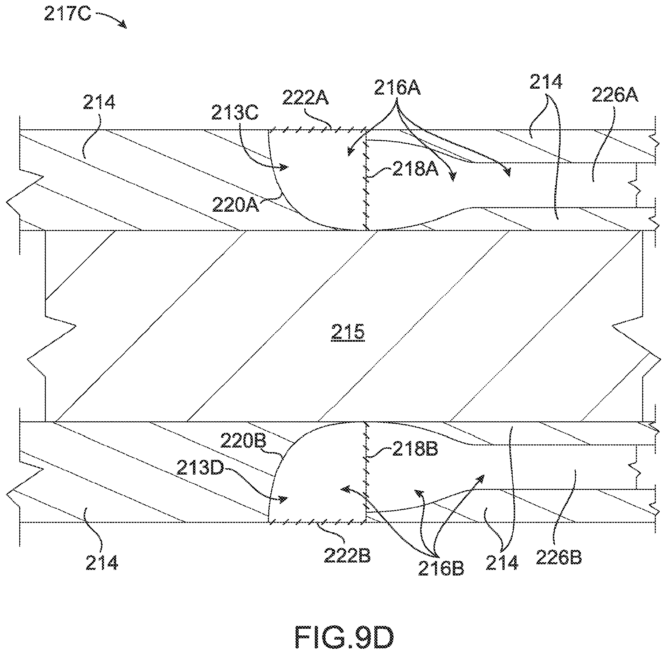

FIGs. 9A-D are identical to FIGs. 4A-D with the exception that in the embodiment of FIGs. 9A-D, the first and second driveline pressure sensor devices 216A and 216B are disposed within the driveline wall 214, and the wire lumen 228 is omitted. As depicted, the first and second driveline pressure sensor devices 216A and 216B (and corresponding first and second driveline pressure signal lines 226A and 226B) are disposed at opposing ends of the driveline wall 214. To differentiate the embodiments, the embodiment of FIGs. 9A-D includes an IABP assembly 900, expandable member 202C and a driveline 204G.

FIGs. 10A-D are identical to FIGs. 2A-D with the exception that an expandable member pressure sensor device is added to the IABP assembly 1000. The expandable member pressure sensor device 1002 is configured to generate an expandable member pressure signal communicative of (e.g., it may indicate or be representative of) the pressure of the environment external to the expandable member 200D. Expandable member pressure signal may be a light wave, a fluid, an electric analog or digital signal, or any other media that is capable of communicating pressure information. The expandable member pressure sensor device 1002 may include an expandable member pressure sensing element 119 that is sensitive to changes in ambient pressure, and is disposed within the expandable member and positioned at or proximate to the distal end 206 of the expandable member 202D. The expandable member pressure sensor device 1002 may further include an expandable member pressure signal line 123 in communication with the expandable member sensing element 119 may be configured to transmit the expandable member pressure signal. A first portion of the expandable member sensing element 123A may be disposed within the expandable member 202D and a second portion of the expandable member sensing element 123B may be disposed within the driveline 204H. Other arrangements of the first driveline pressure sensor device 216A and expandable member pressure sensor device 1002 are expressly contemplated and within the scope of the present disclosure. For example, either or both of the first driveline pressure sensor device 216A and expandable member pressure signal line 213B may be disposed within the driveline wall 214. In another embodiment, a second driveline pressure sensor device 216B in accordance with any of the foregoing embodiments may be included.

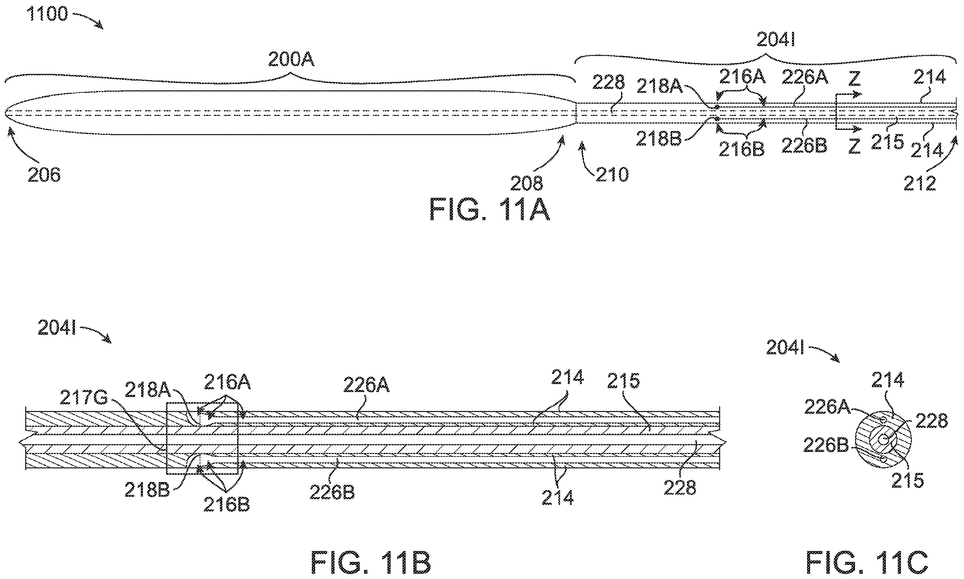

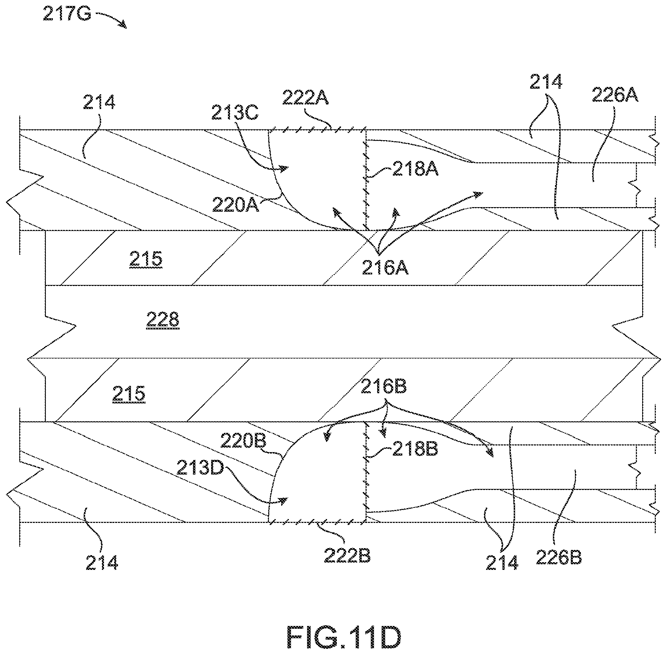

FIGs.11A-D are identical to FIGs. 4A-D with the exception that each of the first and second driveline pressure sensor devices 216A and 216B (and corresponding first and second driveline pressure sensor lines 226A and 226B) are disposed within the driveline wall 214. To differentiate embodiments, FIGs. 11A-D includes an IABP assembly 1100, expandable member 202A and a driveline 204H. In one embodiment, the first and second driveline pressure sensor devices 216A and 216B may be disposed proximate to opposing ends of the driveline wall 214. Other arrangements of the first and second driveline pressure sensor devices 216A and 216B are expressly contemplated and within the scope of the present disclosure.

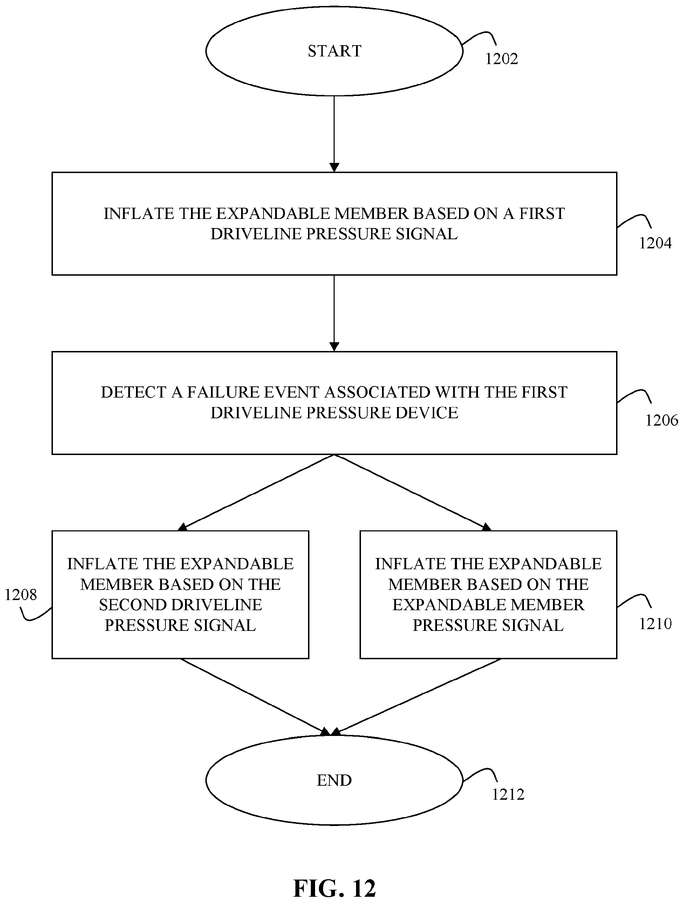

FIG. 12 illustrates a method of using an IABP assembly in accordance with a tenth embodiment of the present disclosure. The method may start at block 1202 where an intra-aortic balloon pump assembly in accordance with any of the embodiments described herein is provided or obtained. The method continues at block 1204 where an expandable member associated with the IABP (e.g., expandable member 202A of FIG. 2A) is inflated based on a first driveline pressure signal (e.g., associated with a first driveline pressure device 216A and communicative of (e.g., it may indicate or be representative of) the pressure of the environment external to the driveline). The method then proceeds to block 1206 where a failure event is detected associated with the first driveline pressure device (e.g., device 216A). The failure device may include a failure to receive a pressure signal, a determination that the pressure signal conveys information that is not expected or anticipated, or some other failure event. In one embodiment, the determination may be made by a computer or processor associated with the IABP assembly (not depicted). The computer or processor may be physically located on the drive unit. The method then proceeds with block 1208 or 1210 where after the detection of the failure event, the expandable member is inflated based on the second driveline pressure signal (e.g., associated with a second driveline pressure device 216B) or an expandable member pressure signal (e.g., associated with an expandable member pressure device 1002). The method then ends in block 1212.

In another embodiment, inflation of the expandable member base be based on both the first and second driveline pressure signals. Where both driveline pressure signals are in agreement (and otherwise not communicating information that is not expected or anticipate), no failure event is determined. However, where the driveline pressure signals are not in an agreement, a failure event may be created. For example, creation of a failure event may include the implementation of an alarm. The method may then proceed with tracking one of the driveline pressure signals to the exclusion of the other. For example, an algorithm (e.g., a voting algorithm) may be implemented to select one over the other based on predetermined criteria. Or the method may be responsive to manual input that selects one driveline pressure signal to the exclusion of the other for purposes of balloon inflation.

### Conclusion

The above detailed description of embodiments of the technology are not intended to be exhaustive or to limit the technology to the precise forms disclosed above. Although specific embodiments of, and examples for, the technology are described above for illustrative purposes, various equivalent modifications are possible within the scope of the technology as those skilled in the relevant art will recognize. The various embodiments described herein may also be combined to provide further embodiments. For example, the above embodiments are not mutually exclusive and the features depicted in any such embodiment may be combined with features of other embodiments. The location/position of the wire lumen 228 may be varied and the number of and relative location/position of the driveline pressure sensor devices may be varied. Similarly, one or more expandable member pressure sensor devices may be added to any embodiment. One of skill in the art will further recognize that the driveline sensing element and associated driveline pressure signal line may be positioned differently (e.g., in the driveline wall, in the working fluid lumen, or in both).

From the foregoing, it will be appreciated that specific embodiments of the technology have been described herein for purposes of illustration, but well-known structures and functions have not been shown or described in detail to avoid unnecessarily obscuring the description of the embodiments of the technology. For example, in Figures 2B, 2D, 4B, 4D,5B, 5D, 6B, 7B, 8B, 9B, 9D, 10B, 10D, 11B, 11D, elements 218 A and 222B and elements 218B and 222B may be one-and-the same. That is, the driveline sensing element may also serve as the driveline sensor window.

Where the context permits, singular or plural terms may also include the plural or singular term, respectively. As used herein, the phrase "and/or" as in "A and/or B" refers to A alone, B alone, and A and B. Additionally, the term "comprising" is used throughout to mean including at least the recited feature(s) such that any greater number of the same feature and/or additional types of other features are not precluded. Further, any ranges identified herein are intended to be inclusive of the numbers that define the range, whether expressly stated or not. The same applies to approximate ranges. It will also be appreciated that specific embodiments have been described herein for purposes of illustration, but that various modifications may be made without deviating from the technology. Further, while advantages associated with some embodiments of the technology have been described in the context of those embodiments, other embodiments may also exhibit such advantages, and not all embodiments need necessarily exhibit such advantages to fall within the scope of the technology. Accordingly, the disclosure and associated technology may encompass other embodiments not expressly shown or described herein.

From the foregoing, it will be appreciated that specific embodiments of the invention have been described herein for purposes of illustration, but that various modifications may be made without deviating from the scope of the invention. Accordingly, the invention is not limited except as by the appended claims.

**By** disposing one or more driveline pressure sensor devices (216A-216B) in the driveline 204A-204I, the pressure of the environment 302 external to and proximate to the distal end 210 of the driveline 204A-204I may be ascertained when in operation. This solves a problem associated with subclavian implantation of IABP assemblies. In particular, it minimizes time delays associated with and increases accuracy of inflation timing in IABPs implanted using an axillary implantation. Preferred, non-limiting embodiments of the present invention will now be described by way of reference to the clauses below.
1. An intra-aortic balloon pump assembly comprising:
   an expandable member having a distal end and a proximal end, the expandable member configured to be positioned in a patient's descending aorta and to provide circulatory support to the patient;
   a driveline having a distal end and a proximal end, the distal end of the driveline configured to be coupleable to the proximal end of the expandable member; and
   a first driveline pressure sensor device disposed within the driveline and configured to generate a first driveline pressure signal communicative of the pressure of the environment external to the driveline, wherein the first driveline pressure sensor device comprises a first driveline sensing element that is sensitive to changes in ambient pressure and positioned at or proximate to the distal end of the driveline.
2. The intra-aortic balloon pump assembly of clause 1, wherein:
   the first driveline pressure sensor device further comprises a first driveline sensor cavity and a first driveline sensor window,
   the first driveline sensing element defines at least a portion of a wall of the first driveline sensor cavity,
   the driveline wall includes a first recess extending inward relative to the exterior of the driveline, the first recess having a shape that at least partially houses the first driveline sensor cavity, and
   the first driveline sensor window is disposed along the first recess and closes the first driveline sensor cavity.
3. The intra-aortic balloon pump assembly of clause 1, wherein:
   the driveline includes a driveline wall, and
   the first driveline pressure sensing element is disposed within the driveline wall.
4. The intra-aortic balloon pump assembly of clause 1, wherein:
   the driveline comprises a working fluid lumen configured to transport a working fluid for at least one of inflation and deflation of the expandable member, and
   the first driveline pressure sensing element is disposed within the working fluid lumen.
5. The intra-aortic balloon pump assembly of clause 1, wherein:
   the driveline includes a driveline wall,
   the driveline comprises a working fluid lumen configured to transport a working fluid for at least one of inflation and deflation of the expandable member, and
   the first driveline pressure sensing element is disposed partially within the driveline wall and partially within the working fluid lumen.
6. The intra-aortic balloon pump assembly of clause 1, wherein the first driveline sensing element is a diaphragm.
7. The intra-aortic balloon pump assembly of clause 2, wherein the first driveline sensor device comprises a first flexible substance disposed in the first driveline sensor cavity, the first flexible substance operative to communicate pressure to the first driveline sensing element.
8. The intra-aortic balloon pump assembly of clause 7, wherein the first flexible substance is one or more of a gel, a fluid, a gas, an elastomer.
9. The intra-aortic balloon pump assembly of clause 7, wherein the first driveline sensor window is configured to prevent the first flexible substance from leaking out of the first driveline sensor cavity when in operation.
10. The intra-aortic balloon pump assembly of clause 2, wherein the first driveline sensor window is sized and shaped to plug the first recess of the driveline wall.
11. The intra-aortic balloon pump assembly of clause 1, wherein the first driveline pressure sensor device:
   further comprises a first driveline pressure signal line in communication with the first driveline sensing element,
   is disposed within the driveline, and
   is configured to transmit the first driveline pressure signal.
12. The intra-aortic balloon pump assembly of clause 11, wherein the first driveline pressure signal line is one of a fiber optic line and a fluid-filled line.
13. The intra-aortic balloon pump assembly of clause 11, wherein a distal end of the first driveline pressure signal line is coupled to the first driveline sensing element and a proximal end of the first driveline pressure signal line is configured to be coupled to a processing module configured to control a drive unit configured to inflate the expandable member.
14. The intra-aortic balloon pump assembly of clause 11, wherein the driveline comprises a working fluid lumen configured to transport a working fluid for at least one of inflation and deflation of the expandable member.
15. The intra-aortic balloon pump assembly of clause 14, wherein the driveline comprises a driveline wall that defines the working fluid lumen.
16. The intra-aortic balloon pump assembly of clause 14, wherein the working fluid is ambient air.
17. The intra-aortic balloon pump assembly of clause 14, wherein the first driveline pressure signal line is one of:
   disposed within the driveline wall,
   disposed within the working fluid lumen, and
   partially disposed within the driveline wall and partially disposed within the working fluid lumen.
18. The intra-aortic balloon pump assembly of clause 17, wherein the driveline comprises a wire lumen configured as a guidewire rail and is sized such that a guidewire may be threaded through said wire lumen.
19. The intra-aortic balloon pump assembly of clause 18, wherein the wire lumen is one of:
   disposed within the driveline wall,
   disposed within the working fluid lumen, and
   partially disposed within the driveline wall and partially disposed within the working fluid lumen.
20. The intra-aortic balloon pump assembly of clause 11, comprising a second driveline pressure sensor device disposed within the driveline and configured to generate a second driveline pressure signal communicative of the pressure of an environment external to the driveline, wherein the second driveline pressure sensor device comprises a second driveline sensing element that is sensitive to changes in ambient pressure and positioned at or proximate to the distal end of the driveline.
21. The intra-aortic balloon pump assembly of clause 20, wherein:
   the second driveline pressure sensor device comprises a second driveline sensor cavity, a second driveline sensor window, and a second driveline pressure signal line,
   the second driveline sensing element defines at least a portion of a wall of the second driveline sensor cavity,
   the driveline wall includes a second recess extending inward relative to the exterior of the driveline, the second recess having a shape that at least partially houses the second driveline sensor cavity,
   the second driveline sensor window is disposed along the second recess and closes the second driveline sensor cavity, and
   the second driveline pressure signal line is in communication with the second driveline sensing element, is disposed within the driveline, and is configured to transmit the second driveline pressure signal.
22. The intra-aortic balloon pump assembly of clause 1, further comprising an expandable member pressure sensor device configured to generate an expandable member pressure signal communicative of the pressure of the environment external to the expandable member, wherein the expandable member pressure sensor device comprises an expandable member pressure sensing element that is sensitive to changes in ambient pressure and disposed within the expandable member and positioned at or proximate to the distal end of the expandable member.
23. The intra-aortic balloon pump assembly of clause 22, wherein the expandable member pressure sensor device further comprises an expandable member pressure signal line in communication with the expandable member sensing element, wherein:
   the expandable member pressure signal line is configured to transmit the expandable member pressure signal,
   a first portion of the expandable member pressure signal line is disposed within the expandable member, and
   when the expandable member is coupled to the driveline, a second portion of expandable member pressure signal line is disposed within the driveline.
24. An intra-aortic balloon pump assembly comprising:
   an expandable member having a distal end and a proximal end, the expandable member configured to be positioned in a patient's descending aorta and to provide circulatory support to the patient;
   a driveline having a driveline wall, a distal end and a proximal end, the distal end of the driveline coupleable to the proximal end of the expandable member;
   a working fluid lumen defined by the driveline wall, the working fluid lumen configured to transport a working fluid for at least one of inflation and deflation of the expandable member;
   a first driveline pressure sensor device disposed within the driveline and configured to generate a first driveline pressure signal communicative of the pressure of the environment external to the driveline,
   an expandable member pressure sensor device configured to generate an expandable member pressure signal communicative of the pressure of the environment external to the expandable member, wherein the expandable member pressure sensor device comprises an expandable member pressure sensing element that is sensitive to changes in ambient pressure and disposed within the expandable member and positioned at or proximate to the distal end of the expandable member.
   wherein:
      the first driveline pressure sensor device comprises a first driveline sensing element, a first driveline sensor cavity, a first driveline sensor window, and a first driveline pressure signal line,
      the first driveline sensing element is sensitive to changes in ambient pressure, is positioned at or proximate to the distal end of the driveline, and defines at least a portion of a wall of the first driveline sensor cavity,
      the driveline wall includes a first recess extending inward relative to the exterior of the driveline, the first recess having a shape that at least partially houses the first driveline sensor cavity,
      the first driveline sensor window is disposed along the first recess and closes the first driveline sensor cavity,
      the first driveline pressure sensing element is disposed within one of the driveline wall and the working fluid lumen, and
      the first driveline pressure signal line is disposed within one of the driveline wall and the working fluid lumen, is in communication with the first driveline sensing element, and is configured to transmit the first driveline pressure signal.
25. The intra-aortic balloon pump assembly of clause 24, wherein:
   the driveline comprises a wire lumen configured as a guidewire rail and is sized such that a guidewire may be threaded through said wire lumen, and
   the wire lumen is disposed within one of the driveline wall and the working fluid lumen.
26. A method of controlling an intra-aortic balloon pump assembly, wherein the intra-aortic balloon pump assembly comprises:
   an expandable member having a distal end and a proximal end, the expandable member positioned in a patient's descending aorta and to provide circulatory support to the patient,
   a driveline having a driveline wall, a distal end and a proximal end, the distal end of the driveline coupled to the proximal end of the expandable member,
   a working fluid lumen defined by the driveline wall, the working fluid lumen configured to transport a working fluid for at least one of inflation and deflation of the expandable member, and
   a first driveline pressure sensor device disposed within the driveline and configured to generate a first driveline pressure signal communicative of the pressure of the environment external to the driveline, wherein the first driveline pressure sensor device comprises a first driveline sensing element that is sensitive to changes in ambient pressure and positioned at or proximate to the distal end of the driveline, and
   a second driveline pressure sensor device disposed within the driveline and configured to generate a second driveline pressure signal communicative of the pressure of the environment external to the driveline, wherein the second driveline pressure sensor device comprises a second driveline sensing element that is sensitive to changes in ambient pressure and positioned at or proximate to the distal end of the driveline,
   the method comprising:
      inflating the expandable member based on the first driveline pressure signal;
      detecting a failure event associated with the first driveline pressure device; and
      inflating the expandable member based on the second driveline pressure signal after the failure event is detected.
27. A method of controlling an intra-aortic balloon pump assembly, wherein the intra-aortic balloon pump assembly comprises:
   an expandable member having a distal end and a proximal end, the expandable member positioned in a patient's descending aorta and to provide circulatory support to the patient,
   a driveline having a driveline wall, a distal end and a proximal end, the distal end of the driveline coupled to the proximal end of the expandable member,
   a working fluid lumen defined by the driveline wall, the working fluid lumen configured to transport a working fluid for at least one of inflation and deflation of the expandable member,
   a first driveline pressure sensor device disposed within the driveline and configured to generate a first driveline pressure signal communicative of the pressure of the environment external to the driveline, wherein the first driveline pressure sensor device comprises a first driveline sensing element that is sensitive to changes in ambient pressure and positioned at or proximate to the distal end of the driveline, and
   an expandable member pressure sensor device configured to generate an expandable member pressure signal communicative of the pressure of the environment external to the expandable member, wherein the expandable member pressure sensor device comprises an expandable member pressure sensing element that is sensitive to changes in ambient pressure and disposed within the expandable member and positioned at or proximate to the distal end of the expandable member,
   wherein the method comprises:
      inflating the expandable member based on the first driveline pressure signal;
      detecting a failure event associated with the first driveline pressure sensor device; and
      inflating the expandable member based on the expandable member pressure signal after the failure event is detected.

### ADDITIONALLY PREFERRED, NON-LIMITING EMBODIMENTS OF THE PRESENT INVENTION WILL NOW BE DESCRIBED BY WAY OF REFERENCE TO THE CLAUSES BELOW

1. An intra-aortic balloon pump assembly comprising:
   an expandable member having a distal end and a proximal end, the expandable member configured to be positioned in a patient's descending aorta and to provide circulatory support to the patient;
   a driveline having a distal end and a proximal end, the distal end of the driveline configured to be coupleable to the proximal end of the expandable member; and
   a first driveline pressure sensor device disposed within the driveline and configured to generate a first driveline pressure signal communicative of the pressure of the environment external to the driveline, wherein the first driveline pressure sensor device comprises a first driveline sensing element that is sensitive to changes in ambient pressure and positioned at or proximate to the distal end of the driveline.
2. The intra-aortic balloon pump assembly of clause 1, wherein:
   the first driveline pressure sensor device further comprises a first driveline sensor cavity and a first driveline sensor window,
   the first driveline sensing element defines at least a portion of a wall of the first driveline sensor cavity,
   the driveline wall includes a first recess extending inward relative to the exterior of the driveline, the first recess having a shape that at least partially houses the first driveline sensor cavity, and
   the first driveline sensor window is disposed along the first recess and closes the first driveline sensor cavity.
3. The intra-aortic balloon pump assembly of clause 1 or 2, wherein:
   the driveline includes a driveline wall, and
   the first driveline pressure sensing element is disposed within the driveline wall.
4. The intra-aortic balloon pump assembly of any one of clauses 1, 2 or 3, wherein:
   the driveline comprises a working fluid lumen configured to transport a working fluid for at least one of inflation and deflation of the expandable member, and
   the first driveline pressure sensing element is disposed within the working fluid lumen.
5. The intra-aortic balloon pump assembly of any one of clauses 1, 2, 3 or 4, wherein:
   the driveline includes a driveline wall,
   the driveline comprises a working fluid lumen configured to transport a working fluid for at least one of inflation and deflation of the expandable member, and
   the first driveline pressure sensing element is disposed partially within the driveline wall and partially within the working fluid lumen.
6. The intra-aortic balloon pump assembly of any one of the preceding clauses, wherein the first driveline sensing element is a diaphragm.
7. The intra-aortic balloon pump assembly of clause 1, 2, 3, 4, 5 or 6, wherein the first driveline sensor device comprises a first flexible substance disposed in the first driveline sensor cavity, the first flexible substance operative to communicate pressure to the first driveline sensing element.
8. The intra-aortic balloon pump assembly of clause 7, wherein the first flexible substance is one or more of a gel, a fluid, a gas, an elastomer.
9. The intra-aortic balloon pump assembly of clause 7 or 8, wherein the first driveline sensor window is configured to prevent the first flexible substance from leaking out of the first driveline sensor cavity when in operation.
10. The intra-aortic balloon pump assembly of any one of clauses 1, 2, 3, 4, 5, 6, 7, 8, 9, or 10, wherein the first driveline sensor window is sized and shaped to plug the first recess of the driveline wall.
11. The intra-aortic balloon pump assembly of any one of the preceding clauses, wherein the first driveline pressure sensor device:
   further comprises a first driveline pressure signal line in communication with the first driveline sensing element,
   is disposed within the driveline, and
   is configured to transmit the first driveline pressure signal.
12. The intra-aortic balloon pump assembly of any one of clauses 1 to 11, wherein the first driveline pressure signal line is one of a fiber optic line and a fluid-filled line.
13. The intra-aortic balloon pump assembly of any one of clauses 1 to 12, wherein a distal end of the first driveline pressure signal line is coupled to the first driveline sensing element and a proximal end of the first driveline pressure signal line is configured to be coupled to a processing module configured to control a drive unit configured to inflate the expandable member.
14. The intra-aortic balloon pump assembly of any one of clauses 1 to 13, wherein the driveline comprises a working fluid lumen configured to transport a working fluid for at least one of inflation and deflation of the expandable member.
15. The intra-aortic balloon pump assembly of any one of clauses 1 to 14, wherein the driveline comprises a driveline wall that defines the working fluid lumen.
16. The intra-aortic balloon pump assembly of any one of clauses 1 to 15, wherein the working fluid is ambient air.
17. The intra-aortic balloon pump assembly of any one of clauses 1 to 16, wherein the first driveline pressure signal line is one of:
   disposed within the driveline wall,
   disposed within the working fluid lumen, and
   partially disposed within the driveline wall and partially disposed within the working fluid lumen.
18. The intra-aortic balloon pump assembly of any one of clauses 1 to 17, wherein the driveline comprises a wire lumen configured as a guidewire rail and is sized such that a guidewire may be threaded through said wire lumen.
19. The intra-aortic balloon pump assembly of any one of clauses 1 to 18, wherein the wire lumen is one of:
   disposed within the driveline wall,
   disposed within the working fluid lumen, and
   partially disposed within the driveline wall and partially disposed within the working fluid lumen.
20. The intra-aortic balloon pump assembly of any one of clauses 1 to 19, comprising a second driveline pressure sensor device disposed within the driveline and configured to generate a second driveline pressure signal communicative of the pressure of an environment external to the driveline, wherein the second driveline pressure sensor device comprises a second driveline sensing element that is sensitive to changes in ambient pressure and positioned at or proximate to the distal end of the driveline.
21. The intra-aortic balloon pump assembly of any one of clauses 1 to 20, wherein:
   the second driveline pressure sensor device comprises a second driveline sensor cavity, a second driveline sensor window, and a second driveline pressure signal line,
   the second driveline sensing element defines at least a portion of a wall of the second driveline sensor cavity,
   the driveline wall includes a second recess extending inward relative to the exterior of the driveline, the second recess having a shape that at least partially houses the second driveline sensor cavity,
   the second driveline sensor window is disposed along the second recess and closes the second driveline sensor cavity, and
   the second driveline pressure signal line is in communication with the second driveline sensing element, is disposed within the driveline, and is configured to transmit the second driveline pressure signal.
22. The intra-aortic balloon pump assembly of any one of clauses 1 to 21, further comprising an expandable member pressure sensor device configured to generate an expandable member pressure signal communicative of the pressure of the environment external to the expandable member, wherein the expandable member pressure sensor device comprises an expandable member pressure sensing element that is sensitive to changes in ambient pressure and disposed within the expandable member and positioned at or proximate to the distal end of the expandable member.
23. The intra-aortic balloon pump assembly of any one of clauses 1 to 22, wherein the expandable member pressure sensor device further comprises an expandable member pressure signal line in communication with the expandable member sensing element, wherein:
   the expandable member pressure signal line is configured to transmit the expandable member pressure signal,
   a first portion of the expandable member pressure signal line is disposed within the expandable member, and
   when the expandable member is coupled to the driveline, a second portion of expandable member pressure signal line is disposed within the driveline.
24. An intra-aortic balloon pump assembly comprising:
   an expandable member having a distal end and a proximal end, the expandable member configured to be positioned in a patient's descending aorta and to provide circulatory support to the patient;
   a driveline having a driveline wall, a distal end and a proximal end, the distal end of the driveline coupleable to the proximal end of the expandable member;
   a working fluid lumen defined by the driveline wall, the working fluid lumen configured to transport a working fluid for at least one of inflation and deflation of the expandable member;
   a first driveline pressure sensor device disposed within the driveline and configured to generate a first driveline pressure signal communicative of the pressure of the environment external to the driveline,
   an expandable member pressure sensor device configured to generate an expandable member pressure signal communicative of the pressure of the environment external to the expandable member, wherein the expandable member pressure sensor device comprises an expandable member pressure sensing element that is sensitive to changes in ambient pressure and disposed within the expandable member and positioned at or proximate to the distal end of the expandable member.
   wherein:
      the first driveline pressure sensor device comprises a first driveline sensing element, a first driveline sensor cavity, a first driveline sensor window, and a first driveline pressure signal line,
      the first driveline sensing element is sensitive to changes in ambient pressure, is positioned at or proximate to the distal end of the driveline, and defines at least a portion of a wall of the first driveline sensor cavity,
      the driveline wall includes a first recess extending inward relative to the exterior of the driveline, the first recess having a shape that at least partially houses the first driveline sensor cavity,
      the first driveline sensor window is disposed along the first recess and closes the first driveline sensor cavity,
      the first driveline pressure sensing element is disposed within one of the driveline wall and the working fluid lumen, and
      the first driveline pressure signal line is disposed within one of the driveline wall and the working fluid lumen, is in communication with the first driveline sensing element, and is configured to transmit the first driveline pressure signal.
25. The intra-aortic balloon pump assembly of clause 24, wherein:
   the driveline comprises a wire lumen configured as a guidewire rail and is sized such that a guidewire may be threaded through said wire lumen, and
   the wire lumen is disposed within one of the driveline wall and the working fluid lumen.
26. A method of controlling an intra-aortic balloon pump assembly, wherein the intra-aortic balloon pump assembly comprises:
   an expandable member having a distal end and a proximal end, the expandable member positioned in a patient's descending aorta and to provide circulatory support to the patient,
   a driveline having a driveline wall, a distal end and a proximal end, the distal end of the driveline coupled to the proximal end of the expandable member,
   a working fluid lumen defined by the driveline wall, the working fluid lumen configured to transport a working fluid for at least one of inflation and deflation of the expandable member, and
   a first driveline pressure sensor device disposed within the driveline and configured to generate a first driveline pressure signal communicative of the pressure of the environment external to the driveline, wherein the first driveline pressure sensor device comprises a first driveline sensing element that is sensitive to changes in ambient pressure and positioned at or proximate to the distal end of the driveline, and
   a second driveline pressure sensor device disposed within the driveline and configured to generate a second driveline pressure signal communicative of the pressure of the environment external to the driveline, wherein the second driveline pressure sensor device comprises a second driveline sensing element that is sensitive to changes in ambient pressure and positioned at or proximate to the distal end of the driveline,
   the method comprising:
      inflating the expandable member based on the first driveline pressure signal;
      detecting a failure event associated with the first driveline pressure device; and
      inflating the expandable member based on the second driveline pressure signal after the failure event is detected.
27. A method of controlling an intra-aortic balloon pump assembly, wherein the intra-aortic balloon pump assembly comprises:
   an expandable member having a distal end and a proximal end, the expandable member positioned in a patient's descending aorta and to provide circulatory support to the patient,
   a driveline having a driveline wall, a distal end and a proximal end, the distal end of the driveline coupled to the proximal end of the expandable member,
   a working fluid lumen defined by the driveline wall, the working fluid lumen configured to transport a working fluid for at least one of inflation and deflation of the expandable member,
   a first driveline pressure sensor device disposed within the driveline and configured to generate a first driveline pressure signal communicative of the pressure of the environment external to the driveline, wherein the first driveline pressure sensor device comprises a first driveline sensing element that is sensitive to changes in ambient pressure and positioned at or proximate to the distal end of the driveline, and
   an expandable member pressure sensor device configured to generate an expandable member pressure signal communicative of the pressure of the environment external to the expandable member, wherein the expandable member pressure sensor device comprises an expandable member pressure sensing element that is sensitive to changes in ambient pressure and disposed within the expandable member and positioned at or proximate to the distal end of the expandable member,
   wherein the method comprises:
      inflating the expandable member based on the first driveline pressure signal;
      detecting a failure event associated with the first driveline pressure sensor device; and
      inflating the expandable member based on the expandable member pressure signal after the failure event is detected.

## Claims

1. An intra-aortic balloon pump assembly comprising:
an expandable member having a distal end and a proximal end, the expandable member configured to be positioned in a patient's descending aorta and to provide circulatory support to the patient;
a driveline having a distal end and a proximal end, the distal end of the driveline configured to be coupleable to the proximal end of the expandable member; and
a first driveline pressure sensor device disposed within the driveline and configured to generate a first driveline pressure signal communicative of the pressure of the environment external to the driveline, wherein the first driveline pressure sensor device comprises a first driveline sensing element that is sensitive to changes in ambient pressure and positioned at or proximate to the distal end of the driveline.

2. The intra-aortic balloon pump assembly of claim 1, wherein:
the first driveline pressure sensor device further comprises a first driveline sensor cavity and a first driveline sensor window,
the first driveline sensing element defines at least a portion of a wall of the first driveline sensor cavity,
the driveline wall includes a first recess extending inward relative to the exterior of the driveline, the first recess having a shape that at least partially houses the first driveline sensor cavity, and
the first driveline sensor window is disposed along the first recess and closes the first driveline sensor cavity.

3. The intra-aortic balloon pump assembly of claim 1 or 2, wherein:
the driveline includes a driveline wall, and
the first driveline pressure sensing element is disposed within the driveline wall.

4. The intra-aortic balloon pump assembly of any one of claims 1, 2 or 3, wherein:
the driveline comprises a working fluid lumen configured to transport a working fluid for at least one of inflation and deflation of the expandable member, and
the first driveline pressure sensing element is disposed within the working fluid lumen.

5. The intra-aortic balloon pump assembly of any one of claims 1, 2, 3 or 4, wherein:
the driveline includes a driveline wall,
the driveline comprises a working fluid lumen configured to transport a working fluid for at least one of inflation and deflation of the expandable member, and
the first driveline pressure sensing element is disposed partially within the driveline wall and partially within the working fluid lumen.

6. The intra-aortic balloon pump assembly of any one of the preceding claims, wherein the first driveline sensing element is a diaphragm.

7. The intra-aortic balloon pump assembly of claim 1, 2, 3, 4, 5 or 6, wherein the first driveline sensor device comprises a first flexible substance disposed in the first driveline sensor cavity, the first flexible substance operative to communicate pressure to the first driveline sensing element.

8. The intra-aortic balloon pump assembly of claim 7, wherein the first flexible substance is one or more of a gel, a fluid, a gas, an elastomer.

9. The intra-aortic balloon pump assembly of claim 7 or 8, wherein the first driveline sensor window is configured to prevent the first flexible substance from leaking out of the first driveline sensor cavity when in operation.

10. The intra-aortic balloon pump assembly of any one of claims 1, 2, 3, 4, 5, 6, 7, 8, 9, or 10, wherein the first driveline sensor window is sized and shaped to plug the first recess of the driveline wall.

11. The intra-aortic balloon pump assembly of any one of the preceding claims, wherein the first driveline pressure sensor device:
further comprises a first driveline pressure signal line in communication with the first driveline sensing element,
is disposed within the driveline, and
is configured to transmit the first driveline pressure signal.

12. The intra-aortic balloon pump assembly of any one of claims 1 to 11, wherein the first driveline pressure signal line is one of a fiber optic line and a fluid-filled line.

13. The intra-aortic balloon pump assembly of any one of claims 1 to 12, wherein a distal end of the first driveline pressure signal line is coupled to the first driveline sensing element and a proximal end of the first driveline pressure signal line is configured to be coupled to a processing module configured to control a drive unit configured to inflate the expandable member.

14. The intra-aortic balloon pump assembly of any one of claims 1 to 13, wherein the driveline comprises a working fluid lumen configured to transport a working fluid for at least one of inflation and deflation of the expandable member.

15. The intra-aortic balloon pump assembly of any one of claims 1 to 14, wherein the driveline comprises a driveline wall that defines the working fluid lumen.
